# EUROPÄISCHE PATENTSCHRIFT

(11) **EP 3 478 349 B1**
(45) Veröffentlichungstag und Bekanntmachung des Hinweises auf die Patenterteilung: **10.06.2020**
(21) Anmeldenummer: 17735100.4
(22) Anmeldetag: 03.07.2017
(51) Int. Cl.: A61M 15/00

(54) **VORRICHTUNG ZUR AUSGABE EINER DURCH LUFT AUSTRAGBAREN SUBSTANZ**
DEVICE FOR DISPENSING AN AIR-DISCHARGEABLE SUBSTANCE
DISPOSITIF DE DÉLIVRANCE D'UNE SUBSTANCE POUVANT ÊTRE ÉVACUÉE PAR L'AIR

(30) Priorität: 04.07.2016 DE 102016008015; 18.10.2016 DE 102016119789
(43) Veröffentlichungstag der Anmeldung: 08.05.2019
(73) Patentinhaber: Von Schuckmann, Alfred, 47627 Kevelaer (DE)
(72) Erfinder: Von Schuckmann, Alfred, 47627 Kevelaer (DE)
(74) Vertreter: Müller, Enno
(86) Internationale Anmeldenummer: PCT/EP2017/066436
(87) Internationale Veröffentlichungsnummer: WO 2018/007287

(56) Entgegenhaltungen:
- EP-A1- 2 082 764
- WO-A1-00/64779
- WO-A1-2011/071845
- WO-A1-2012/128692
- WO-A1-2015/110832
- WO-A2-01/85097
- WO-A2-2008/053253
- WO-A2-2012/004485
- WO-A2-2014/006135
- DE-A1-102008 023 376
- DE-A1-102009 041 664
- DE-A1-102014 005 646
- GB-A- 2 407 042
- US-A1- 2007 295 333

## Beschreibung

### Gebiet der Technik

Die Erfindung betrifft eine Vorrichtung zur Ausgabe einer durch Luft austragbaren Substanz mit einem Lufteinlass, einer Austragsdüse, durch welche mit Substanz befrachtete Luft austreten kann, einem Entleerungsbereich, in welchem eine Kamme mit auszutragender Substanz in einer Kammeraufnahme angeordnet ist, einem von dem Entleerungsbereich zu der Austragsdüse führenden ersten Luftführungskanal und einem von dem Lufteinlass zu dem Entleerungsbereich führenden zweiten Luftführungskanal..

Eine solche Vorrichtung weist einen Lufteinlass und eine Austragsdüse auf. Die Austragsdüse kann als Mundstück ausgebildet sein, zur Saugbeaufschlagung durch einen menschlichen Benutzer. Durch die Austragsdüse kann die mit Substanz befrachtete Luft, insbesondere infolge einer Saugluftbeaufschlagung, austreten.

Die Vorrichtung dient weiter zur Ausgabe von Substanz, die in einer Kammer vorliegt. Die Kammer kann topfartig, bevorzugt aus einem ggf. auch transparent eingestellten Hartkunststoff bestehend, gestaltet sein. Die Kammer kann eine folienartige, bevorzugt zum Öffnen durchstechbare Abdeckung aufweisen. Die Kammer kann auch eine Blisterkammer sein oder Teil eines Blisterstreifens. Es kann sich um eine Vorrichtung zur Einmalentleerung handeln, wonach die Vorrichtung zu öffnen ist und eine neue Kammer einzusetzen ist, oder um eine Vorrichtung, bei welcher sukzessive, bevorzugt schrittweise, mehrere Kammern in den Entleerungsbereich verbracht werden, etwa vermittels eines Blisterstreifens.

### Stand der Technik

Zum Stand der Technik ist zunächst auf die WO 2010/084415 A1 (US 2012/0037158 A1) zu verweisen, weiter auf die WO 01/26720 A1 (US 6,880,555 B1).

Aus der GB 2 407042 A ist eine Vorrichtung zur Ausgabe einer durch Luft austragbaren Substanz bekannt, bei welcher eine Einströmmündung mit kreisförmiger Randkontur ausgebildet ist. Ein vergleichbarer Stand der Technik ist auch durch die EP 208 2764 A1 gegeben.

Aus der WO2014/006135 A2 ist eine Vorrichtung bekannt, bei welcher der erste Luftführungskanal unter Nutzung einer Oberseite der Kammer, die zu entleeren ist, als ebenflächige Oberfläche im verschlossenen Zustand der Vorrichtung mit sich in Strömungsrichtung erstreckenden Rippen des verschwenkbaren Vorrichtungsteils zusammengeführt wird. Ein vergleichbarer Stand der Technik aus der DE 10 2008 023 376 A1 bekannt. Im Weiteren ist auf die DE 10 2014 005 646 A1, die DE 10 2009 041 664 A1 und die WO2015/110832 A1 zu verweisen.

### Zusammenfassung der Erfindung

Ausgehend von dem dargelegten Stand der Technik beschäftigt sich die Erfindung mit der Aufgabenstellung, eine Vorrichtung zur Ausgabe einer durch Luft austragbaren Substanz im Hinblick auf eine Zusammenwirkung mit der Kammer vorteilhaft auszubilden.

Hierzu ist gemäß dem Anspruch 1 insbesondere darauf abgestellt, dass in dem Entleerungsbereich zwischen dem ersten und dem zweiten Luftführungskanal eine Trennwandung ausgebildet ist und die Trennwandung aus einem Hartwerkstoff besteht, wobei weiter eine der Kammer zugewandte freie Stirnfläche der Trennwandung mit einem Weichwerkstoff versehen ist, die Kammer in einer Überdeckung zu einer Substanz enthaltenden Kammerhöhlung eine Folienabdeckung aufweist und die Trennwandung vermittels dieses Weichwerkstoffes dichtend an der Folienabdeckung anliegt.

Der zweite Luftführungskanal kann t über einen Verbindungskanal mit dem ersten Luftführungskanal mit dem ersten Luftführungskanal in Strömungsverbindung stehen. Der Verbindungskanal bildet einen Bypass, durch welchen die Luft bevorzugt frei von Substanz strömen kann. Die mit Substanz beladbare Luftströmung kann über einen weiteren, im Wesentlichen durch die beiden Verbindungskanäle gebildeten Strömungsweg geführt sein.

Eine Einströmrichtung von durch eine Einströmmündung in dem ersten Luftführungskanal strömender Luft ist senkrecht gerichtet zu einer Strömungsrichtung der mit Substanz befrachteten Luft in dem ersten Luftführungskanal im Bereich der Einströmmündung. Durch die im Bereich der Einströmöffnung aufeinandertreffenden Luftströme erfolgt eine günstige Verwirbelung im Bereich der Austragsdüse. Hierdurch wird ein weiterer Aufschluss der Substanz erreicht, so dass es zu den gewünschten sehr kleinen Partikeln kommen kann, die günstig zu inhalieren sind.

Es kann auch nur ein (Zahlwort) solcher Verbindungskanal vorgesehen sein.

Die bodenseitig vorgesehene Einströmmündung ist als quer zu der Strömungsrichtung der mit Substanz befrachteten Luft ausgebildete schlitzartige Öffnung gestaltet, die sich über einen wesentlichen Teil der Breite, mehr als 50% der quer zur Strömungsrichtung betrachteten Breite, bis hin zu 90 oder 100% der Breite des ersten Luftführungskanals erstreckt. Insbesondere ist bevorzugt gegenüberliegend zu der Einströmmündung die Wandung (Decke) des ersten Luftführungskanals geschlossen ausgebildet.

Der Weichwerkstoff der Trennwandung kann bspw. Gummi oder ein TPE (thermoplastisches Elastomer) sein. Die Stirnfläche kann in Überdeckung zu einer beispielhaften Folienabdeckung der Kammer verlaufen. Die Stirnfläche kann eine Kammerhöhlung in einer Anlagesituation überqueren, so dass in einer gedachten Fortführung der Stirnfläche in die Kammer hinein diese zu einer Teilung der Kammerhöhlung führen würde, wobei sich in jeder solcher Teilkammer dann Substanz befindet. Die Stirnfläche kann an einer Folienabdeckung der Kammer im Benutzungszustand dichtend anliegend.

Die Kammeraufnahme der Vorrichtung kann in einem ersten Gehäuseteil ausgebildet sein und ein zweites Gehäuseteil kann vorgesehen sein, das mit dem ersten Gehäuseteil schwenkbar verbunden ist.

Der Luftführungskanal kann über einen Teil seiner Länge und in seiner Längsrichtung gesehen längsgeteilt ausgebildet sein. Ein erster und ein zweiter Teilbereich des Luftführungskanals sind an dem ersten und an dem zweiten Gehäuseteil ausgebildet. So ergibt sich ein im Querschnitt vollständig geschlossener Luftführungskanal zumindest über einen Teil der Länge erst dann, wenn sich das zweite Gehäuseteil in einer ordnungsgemäßen Nutzungsstellung relativ zu dem ersten Gehäuseteil befindet. Von dem Entleerungsbereich führt ein erster Luftführungskanal zu der Austragsdüse und von dem Lufteinlass ein zweiter Luftführungskanal zu dem Entleerungsbereich. Der erste Luftführungskanal und der zweite Luftführungskanal sind über jedenfalls einen Teil ihrer Länge geteilt ausgebildet, wobei an dem ersten Gehäuseteil ein erster Teilbereich des zweiten Luftführungskanals und ein zweiter Teilbereich des ersten Luftführungskanals und an dem zweiten Gehäuseteil ein dritter Teilbereich des zweiten Luftführungskanals und ein vierter Teilbereich des ersten Luftführungskanals gegeben sind. Die zwei Teilbereiche des zweiten Luftführungskanals und des ersten Luftführungskanals können als für sich gesehen zu einem Luftführungskanal nicht gehörend erkennbare Flächenbereiche einer ansonsten darüber hinausgehenden ebenflächigen Oberfläche gebildet sein. Die weiteren Teilbereiche können kammerartig gebildet sein, bei denen gleichsam im Öffnungszustand ein Deckel oder Boden fehlt und durch die Zusammenwirkung im geschlossenen Zustand sich erst der geschlossene erste und zweite Luftführungskanal ergibt. Der dritte Teilbereich und der vierte Teilbereich sind durch ein Verschwenken des zweiten Gehäuseteils in eine bevorzugt verrastete, jedenfalls aber eine Anlagestellung zu dem ersten Gehäuseteil zu dem jeweiligen ersten beziehungsweise zweiten Luftkanal ergänzbar. Zugleich ist hierbei eine für die Luftströmung undurchlässige Trennwand bei eingesetzter Kammer relativ zu der Kammer in eine Anlagestellung bewegbar und durch die Trennwand eine unmittelbare Luftströmung von dem zweiten Luftführungskanal in den ersten Luftführungskanal unterbindbar, wobei die Luftströmung im Benutzungszustand nur durch die Kammer verläuft.

Die Kammer kann eine durchstechbare Kammerabdeckung, etwa in Form der schon genannten Folienabdeckung, aufweisen. Es kann eine Einstecheinrichtung zur Ausbildung zumindest einer Einstechöffnung in der Kammerwandung vorgesehen sein. Hinsichtlich der bevorzugt in dem Entleerungsbereich vorgesehenen Trennwandung zur Umlenkung von Luft in die Kammer kann weiter vorgesehen sein, dass die Einstecheinrichtung zwei Einstechvorsprünge aufweist, von denen einer zugeordnet der einen Seite der Trennwandung und der andere zugeordnet der anderen Seite der Trennwandung ausgebildet ist. So kann beidseitig bzw. mit Bezug zu der Strömungsrichtung vor und hinter der Trennwandung jeweils eine Einstechöffnung in der Kammerabdeckung vorgesehen werden. Ein Einstechvorsprung ist in einem Querschnitt an eine sich in Niederdrückrichtung gesehen darbietenden Innenkontur einer Kammerwandung angepasst. Die Kammerwandung ist in dem Querschnitt kreisringförmig gebildet und der Einstechvorsprung, in einem die Kammerabdeckung bei einer Verlagerung des Einstechvorsprungs in einer Einstechrichtung durchsetzenden Bereich, weist eine kreisabschnittsförmige Außenkontur auf. Der Einsteckvorsprung weist eine Spitze oder Schneide auf, die im Zuge des Einstechens unmittelbar an einer Innenwand der Kammer entlangfährt.

Der im Zusammenhang vorliegender Anmeldung angesprochene Hartwerkstoff, insbesondere ein Hartkunststoff, kann von einem Weichwerkstoff, insbesondere einem Weichkunststoff, beispielsweise hinsichtlich einer Shore-Härte unterschieden werden, etwa gemessen entsprechend DIN 53505 von 2012. In dieser Hinsicht kann der Hartkunststoff insbesondere eine Shore-Härte aufweisen, die größer als 60 ist, weiter insbesondere größer als 65, beispielsweise eine Shore-Härte in einem Bereich von 75 bis 80.

Weitere Merkmale der Erfindung sind nachstehend, auch in der Figurenbeschreibung, oftmals in ihrer bevorzugten Zuordnung zu den bereits vorstehend erläuterten Anspruchskonzepten beschrieben, sie können aber auch in einer Zuordnung zu nur einem oder mehreren einzelnen Merkmalen, wie hier beschrieben sind, insbesondere der bereits abgehandelten Ansprüche, oder unabhängig oder in einem anderen Gesamtkonzept von Bedeutung sein. Auch ist es möglich, die Maßnahmen der bereits behandelten Ansprüche kombiniert vorzusehen.

So kann sich die Trennwandung mit ihrer freien Stirnfläche in Anlagestellung zu der Folienabdeckung der Kammer quergerichtet zu der Strömungsrichtung im Luftführungskanal erstrecken. Durch die Trennwandung kann eine unmittelbare Luftströmung von dem zweiten Luftführungskanal in den ersten Luftführungskanal unterbunden sein. Dies insbesondere weiter zufolge einer möglichen dichtenden Anlage der Trennwandung auf einer kammerseitigen Abdeckung. Die Trennwandung kann hierbei so angeordnet sein, dass diese quer zur Luftströmungsrichtung betrachtet gegenüberliegende Wandungsbereiche des Luftführungskanals verbindet. Diese Verbindung ist bevorzugt vollständig abdichtend. Da die Trennwandung selbst bevorzugt auch undurchlässig für eine Luftströmung ist, kann die Luftströmung im Benutzungszustand nur durch die Kammer verlaufen.

Die Einstecheinrichtung kann entgegen einer Rückstellkraft verlagerbar sein, zur Durchsetzung der Folienabdeckung der Kammer durch die Einstechvorsprünge. Die Rückstellkraft kann aus dem Material der Einstecheinrichtung resultieren, bspw. bei Ausgestaltung der Einrichtung aus einem rückstellfähigen Material.

In einer weiteren möglichen Ausgestaltung resultiert die Rückstellkraft aus einer Feder. Hierbei kann es sich bei Ausbildung der Einstecheinrichtung aus einem Kunststoffmaterial um eine angespritzte Feder, bspw. einen angespritzten Federarm handeln. Darüber hinaus kann auch ein thermoplastisches Elastomermaterial die entsprechende Rückstellkraft aufbringen. Auch kann diesbezüglich eine übliche Feder, bspw. Stahlfeder, vorgesehen sein, etwa in Form einer Wendelfeder, weiter bspw. eine Zylinder-Druckfeder oder eine Blattfeder oder dergleichen.

In einer weiteren möglichen Ausgestaltung kann die Vorrichtung eine verlagerbare Drucktaste aufweisen, zur Einwirkung auf die Einstecheinrichtung. Die Drucktaste ist bei üblicher Nutzung der Vorrichtung frei von außen zugänglich, bspw. zur Beaufschlagung mit einem oder mehreren Fingern. Die Drucktaste kann schiebeverlagerbar an der Vorrichtung, bspw. an dem zweiten Gehäuseteil gehaltert sein.

Die Drucktaste kann darüber hinaus entgegen der Rückstellkraft bspw. einer Feder unter Mitschleppen der Einstecheinrichtung in Richtung auf die Kammeraufnahme verlagerbar sein.

Nach einem Durchstechen der Folienabdeckung der Kammer durch die Einstechvorsprünge kann die Kammer den ersten Luftführungskanal mit dem zweiten Luftführungskanal strömungsmäßig verbinden. Die Kammer ist nach Durchstechen der Folienabdeckung Teil des gesamten Luftführungskanals und bildet die alleinige Verbindung, mit Ausnahme der Kurzschluss- bzw. Bypass-Verbindung, zwischen den beiden Teil-Luftführungskanälen.

Das zweite Gehäuseteil kann über ein Scharnier mit dem ersten Gehäuseteil unverlierbar verbunden sein. Eine geometrische Scharnierachse kann quergerichtet zur Luftströmungsrichtung ausgerichtet sein. Eine mögliche Umlenkung des zweiten Luftführungskanals kann zugeordnet dem Scharnierbereich ausgebildet sein, wobei weiter der Scharnierbereich bevorzugt in einem der Austragsdüse abgewandten Endbereich der Vorrichtung vorgesehen sein kann.

In Strömungsrichtung nach der Umlenkung kann die Luftströmung entgegengesetzt gerichtet sein zu der Luftströmung vor der Umlenkung. Weiter kann vor der Umlenkung die Luft allein durch den zweiten Luftführungskanal geführt sein und in Luftströmungsrichtung betrachtet nach der Umlenkung durch einen weiteren Teilabschnitt des zweiten Luftführungskanals und über den ersten Luftführungskanal hin zur Austragsdüse.

Die Kammeraufnahme kann darüber hinaus in Strömungsrichtung betrachtet nach der Umlenkung vorgesehen sein.

Ein Einstechvorsprung kann in einem Querschnitt betrachtet, insbesondere einem Querschnitt quer zur Verlagerungsrichtung des Vorsprunges, an eine sich in der Niederdrückrichtung gesehen darbietende Innenkontur einer Kammerwandung angepasst sein. So kann bei einer in einem solchen Querschnitt betrachteten kreisringförmigen Ausgestaltung der Kammerwandung der Einstechvorsprung insbesondere in dem die Kammerabdeckung bei Verlagerung des Einstechvorsprungs in Einstechrichtung durchsetzenden Bereich eine kreisabschnittförmige Außenkontur aufweisen. Diese kreisabschnittförmige Kontur kann sich mit Bezug auf den erzeugenden Kreis in dem genannten Querschnitt auf denselben Mittelpunkt beziehen, wie die kreisringförmige Kontur, insbesondere die Innenkontur der Kammerwandung.

Ein Einstechvorsprung kann auch an seiner einer Mittelachse der Kammer zugewandten Seite in dem Querschnitt kreisabschnittförmig verlaufen. Der Einstechvorsprung kann eine Dicke in diesbezüglich radialer Richtung aufweisen, die einem Zwanzigstel bis einem Fünftel des Innendurchmessers der Kammer in einem Querschnitt, in welchem das Einstechen erfolgt, entspricht.

Eine Erstreckung eines Einstechvorsprunges in einer Umfangsrichtung bezüglich der Mittelachse der Kammer kann sich etwa über ein Zehntel bis ein Drittel der Umfangserstreckung eines in einem Querschnitt, in welchem das Einstechen erfolgt, gegebenen Kreises, der mit der Innenwand der Kammer zusammenfällt, erstrecken.

Ein Einstechvorsprung kann weiter eine Spitze ausbilden, die punktförmig gestaltet sein kann, aber auch im Sinne einer über zumindest einen wesentlichen Teil der Umfangserstreckung des Einstechvorsprunges umlaufende Schneide. Die Spitze oder die Schneide sind darüber hinaus bevorzugt so ausgebildet, dass sie im Zuge des Einstechens unmittelbar an einer Innenwand der Kammer entlang fahren. Entsprechend vergrößert sich bei dieser Ausgestaltung der Einstechvorsprung von der Spitze oder der Schneide aus gesehen nur zum Inneren der Kammer hin. Diesie Vergrößerung kann in Form einer abfallenden Schrägfläche ausgebildet sein derart, dass die Spitze oder Schneide im Zuge des Einstechens den vordersten Bereich bildet.

Durch eine Ausgestaltung mit der Spitze oder der Schneide entlangfahrend unmittelbar an der Innenfläche der Kammer lässt sich erreichen, dass die Abdeckung von der Kammerwandung ausgehend nach innen bezüglich der Kammer aufgetrent wird. Somit ist die Abdeckung an der Kammerwandung praktisch fluchtend mit der Kammerwandung entfernt, dies sowohl auf der Einströmseite wie auf der Ausströmseite. Eine Luftströmung kann insbesondere auch unmittelbar entlang der Kammerwandung über den Boden und wieder an der Kammerwandung hoch erfolgen und somit ein angestrebtes möglichst vollständiges Entleeren der Kammer wirksam unterstützen.

Auch kann die Folienabdeckung der Kammer, in einer Ansicht von außen, weiter bevorzugt in einer Ansicht in Einstechrichtung auf die kammeräußere Seite der Abdeckung, konkav gestaltet sein. Diese so gegebene konkave Einwölbung der Kammerabdeckung kann auch in unbelasteter Stellung der Abdeckung grundsätzlich gegeben sein. In einer möglichen, auch bevorzugten, Ausgestaltung ergibt sich die konkave Gestaltung im eingesetzten Zustand der Kammer und ggf. auch nur dann, dies zufolge Beaufschlagung der Kammerabdeckung durch die Trennwandung. Die Stirnfläche der Trennwandung kann hierzu im abdichtenden Beaufschlagungsbereich angepasst ausgeformt sein, entsprechend zumindest abschnittweise eine konvexe Krümmung bildend.Kurze Beschreibung der Zeichnungen

Nachstehend ist die Erfindung anhand der beigefügten Zeichnung erläutert, die aber lediglich Ausführungsbeispiele darstellt. Ein Teil, das nur bezogen auf eines der Ausführungsbeispiele erläutert ist, und bei einem weiteren Ausführungsbeispiel aufgrund der dort herausgestellten Besonderheit nicht (gerade) durch ein anderes Teil ersetzt ist, ist damit auch für dieses weitere Ausführungsbeispiel als jedenfalls mögliches vorhandenes Teil beschrieben. Hierbei zeigt:
- Fig. 1: eine Seitenansicht der aufgeklappten Vorrichtung in einer ersten Ausführungsform, mit einer in die Vorrichtung einzusetzenden Kammer;
- Fig. 2: eine Ansicht der Vorrichtung gemäß Figur 1, perspektivisch gesehen ohne eingesetzte Kammer;

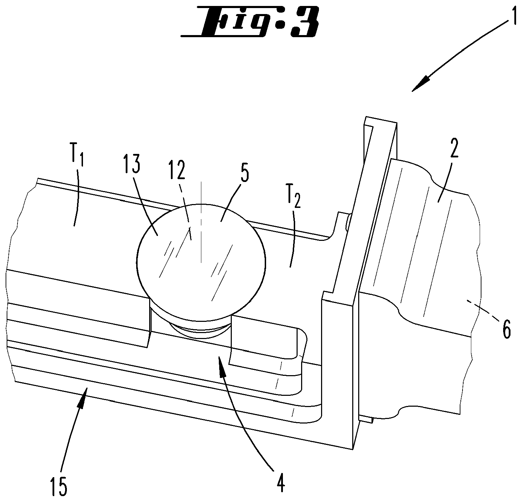

- Fig. 3: eine vergrößerte Darstellung der Vorrichtung gemäß Figur 1 bzw. Figur 2, mit eingesetzter Kammer;
- Fig. 4: einen perspektivischen Längsschnitt durch die Vorrichtung im zusammengesetzten Zustand und mit eingesetzter Kammer;

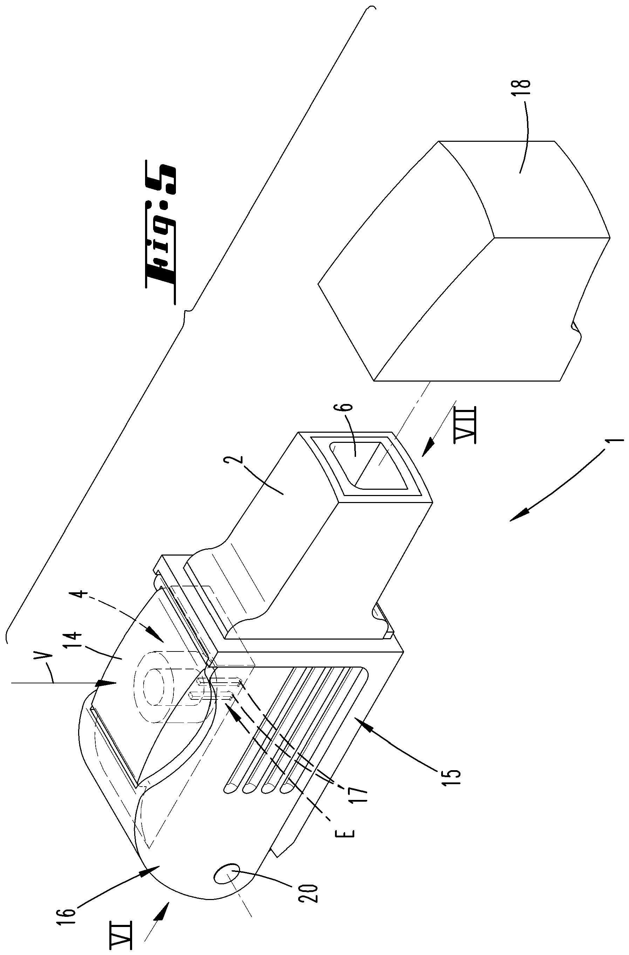

- Fig. 5: eine Darstellung gemäß Figur 4, in einer Außenansicht;
- Fig. 6: die Vorrichtung gemäß Figur 4 bzw. Figur 5 in der Rückansicht gemäß Pfeil VI in Figur 5;
- Fig. 7: die Vorrichtung in Vorderansicht gemäß Pfeil VII in Figur 5;
- Fig. 8: die Vorrichtung gemäß Figur 4 bzw. Figur 5 in einer Seitenansicht;
- Fig. 9: die Vorrichtung gemäß Figur 4 in einer perspektivischen Ansicht von schräg hinten;

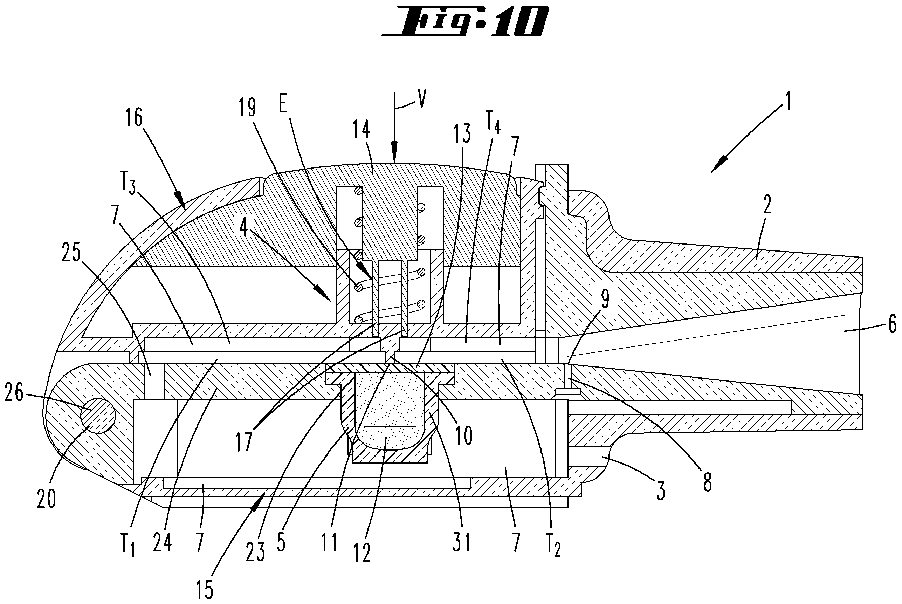

- Fig. 10: einen Querschnitt durch die Vorrichtung gemäß Figur 7, geschnitten entlang der Ebene X-X;
- Fig. 11: eine Darstellung gemäß Figur 4, bei heruntergedrückter Einstechvorrichtung;

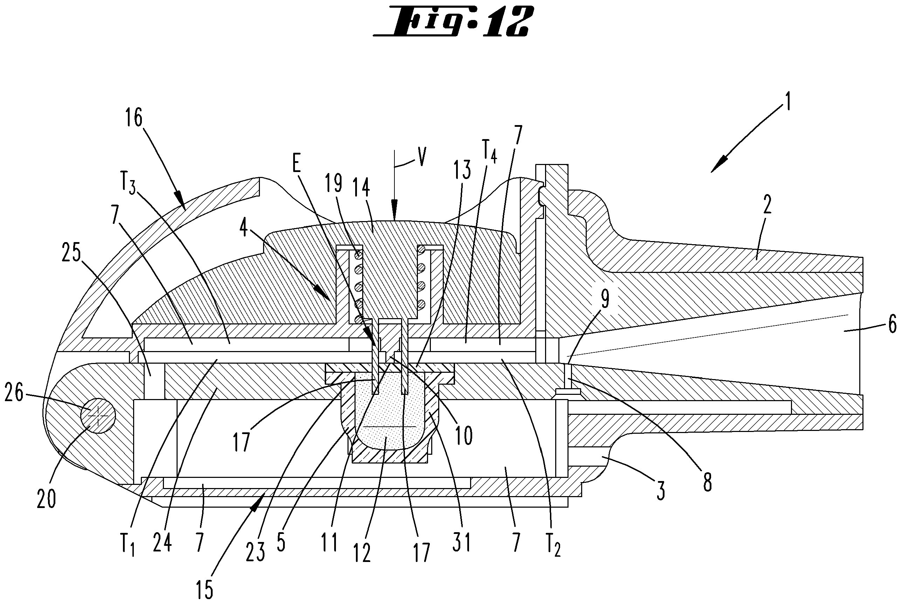

- Fig. 12: eine Darstellung gemäß Figur 10 bei heruntergedrückter Einstechvorrichtung;
- Fig. 13: die Vorrichtung in perspektivischer Darstellung, betreffend eine zweite Ausführungsform;
- Fig. 14: eine der Figur 2 im Wesentlichen entsprechende perspektivische Darstellung, betreffend die Vorrichtung gemäß Figur 13;
- Fig. 15: eine der Figur 3 im Wesentlichen entsprechende Darstellung betreffend die Vorrichtung gemäß den Figuren 13 und 14;
- Fig. 16: die Vorrichtung der zweiten Ausführungsform in perspektivischer Explosionsdarstellung;
- Fig. 17: einen Längsschnitt durch die Vorrichtung gemäß Figur 13;
- Fig. 18: die Herausvergrößerung des Bereiches XVIII in Figur 17;
- Fig. 19: eine der Figur 18 entsprechende Darstellung bei heruntergedrückter Einstechvorrichtung;
- Fig. 20: den Schnitt gemäß der Linie XX-XX in Figur 19;
- Fig. 21: den Schnitt gemäß der Linie XXI-XXI in Figur 20;
- Fig. 22: eine Folgedarstellung zu Figur 19, die Vorrichtung im Zuge eines Inhalationsvorganges betreffend.

### Beschreibung der Ausführungsformen

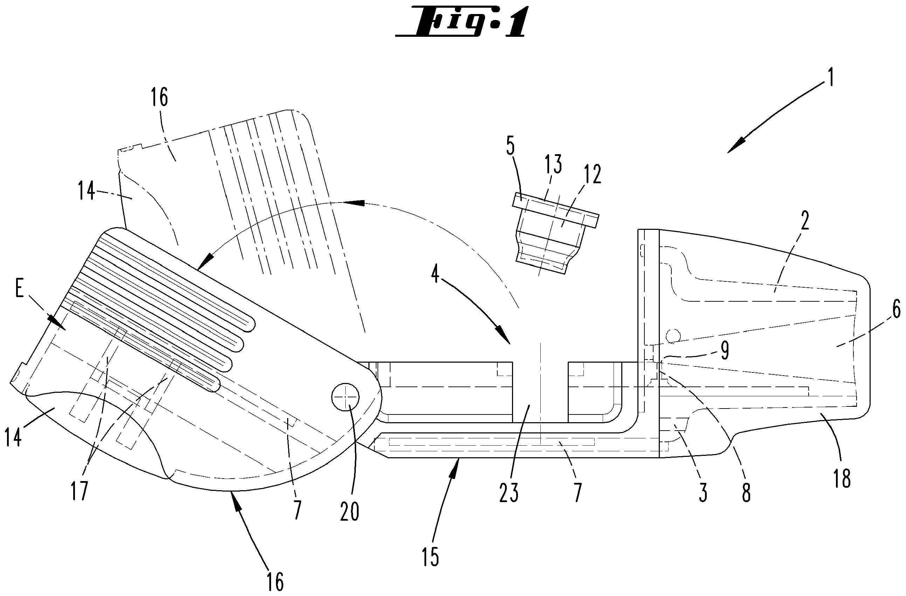

Dargestellt und beschrieben ist eine Vorrichtung 1, siehe etwa Figuren 1, 4 und 7, mit einer als Mundstück ausgebildeten Austragsdüse 2, einem Lufteinlass 3, der bevorzugt durch mehrere nebeneinander angeordnete Bohrungen gebildet ist, und einem Entleerungsbereich 4, in welchem eine mit Substanz gefüllte Kammer 5 einsetzbar ist. Die Substanz, die bevorzugt eine pharmazeutische Substanz ist, ist mittels Luft, etwa über eine von einem Benutzer durch Ansaugen erzeugte Luftströmung, austragbar.

Die Austragsdüse 2 ist in einer Nichtbenutzungsstellung der Vorrichtung 1 mittels einer Kappe 18 überdeckt.

Von dem Entleerungsbereich 4 führt ein erster Luftführungskanal 6 zu der Austragsdüse 2. Von dem Lufteinlass 3 führt ein zweiter Luftführungskanal 7 zu dem Entleerungsbereich 4.

Bevorzugt ist zwischen dem zweiten Luftführungskanal 7 und dem ersten Luftführungskanal 6 ein Verbindungskanal 8 ausgebildet, über welchen der zweite Luftführungskanal 7 mit dem ersten Luftführungskanal 6 in einer Strömungsverbindung ist.

Der Verbindungskanal 8 ist in Strömungsrichtung S betrachtet zumindest annähernd unmittelbar hinter dem Lufteinlass 3 angeordnet. Auch kann der Verbindungskanal 8 im Wurzelbereich der Austragsdüse 2, entsprechend im Übergangsbereich von der Austragsdüse 2 in das übrige Vorrichtungsgehäuse angeordnet sein.

Der Verbindungskanal 8 bildet eine Bypass- oder Kurzschluss-Verbindung neben dem die Substanz austragenden Luftführungsweg.

Durch den Verbindungskanal 8 mit einer Einströmrichtung R in den ersten Luftführungskanal 6 strömende Luft ist im Wesentlichen senkrecht gerichtet zu einer Strömungsrichtung S der mit Substanz befrachteten Luft, die in dem ersten Luftführungskanal 6 im Zustand einer Benutzung strömt.

Diese Strömungsrichtung S ist jedenfalls im Bereich einer Einströmmündung 9 des Verbindungskanals 8 in den ersten Luftführungskanal 6 gegeben.

Bezüglich des Entleerungsbereiches 4 kann in der Vorrichtung 1 eine Trennwandung 10 ausgebildet sein, die bevorzugt, wie im Übrigen die große Mehrzahl der weiteren Teile der Vorrichtung auch, aus einem Hartwerkstoff, wie insbesondere einem Hartkunststoff besteht.

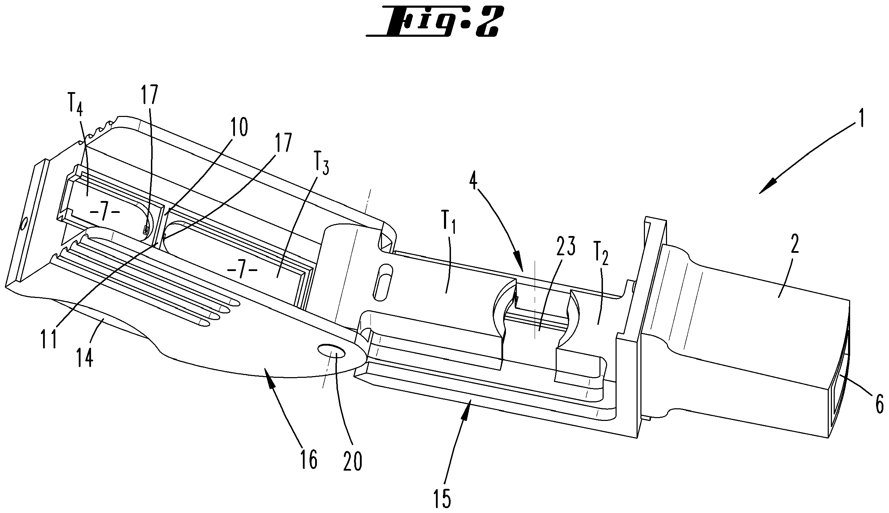

Die Trennwandung 10 bildet eine der Kammer 5 zugewandte Stirnfläche 11 aus, siehe auch Figur 2. Die Stirnfläche 11 kann mit einem Weichwerkstoff versehen sein. Der Weichwerkstoff kann bspw. Gummi oder ein TPE sein.

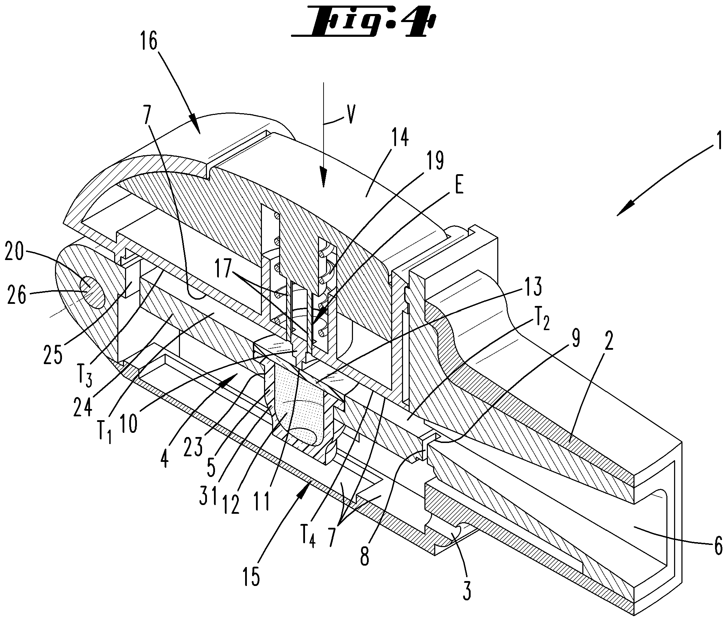

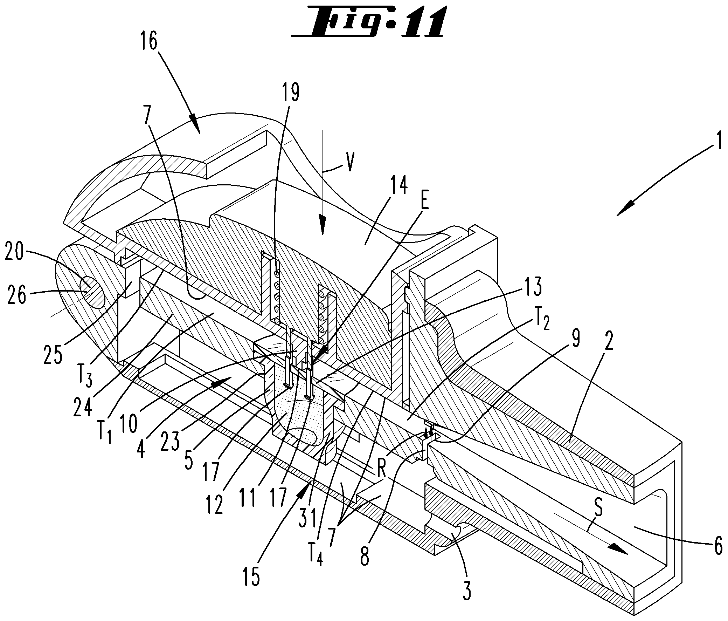

Die Stirnfläche 11 verläuft im Benutzungszustand, wie etwa aus den Figuren 4 und 11 ersichtlich, in einer Überdeckung zu einer Kammerhöhlung 12, in der sich die auszutragende Substanz befindet.

Die Kammerhöhlung 12 weist eine Abdeckung 13, bevorzugt in Form einer Folienabdeckung, auf. Die Abdeckung 13 ist weiter bevorzugt öffenbar, nämlich insbesondere durchstechbar. Im Bereich der, vermittels etwa des Weichwerkstoffes, anliegenden Stirnfläche 11 ist die Abdeckung im Benutzungszustand bevorzugt unversehrt.

Die Stirnfläche 11 der Trennwandung 10 liegt in einem Benutzungszustand etwa gemäß Figur 4, bevorzugt auch vermittels des Weichwerkstoffes, dichtend an der Abdeckung 13 an. Sie überquert die Höhlung 12 der Kammer 5 derart, dass eine Projektion der Trennwandung 10 in Richtung einer Niederdrückrichtung V eines Betätigungselementes 14 die Höhlung 12 der Kammer 4 kreuzend durchsetzt.

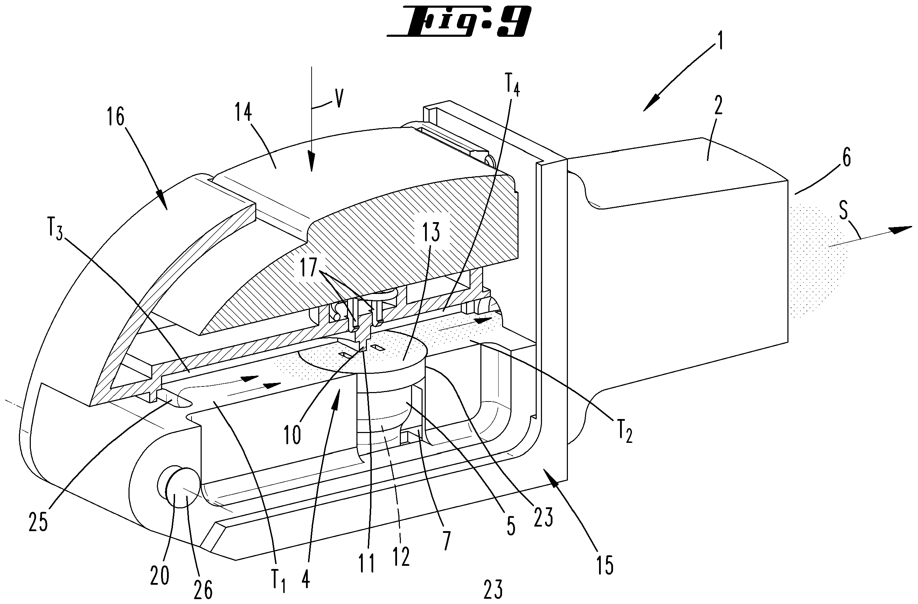

Der erste Luftführungskanal 6 und/oder der zweite Luftführungskanal 7 sind bevorzugt über ihre Länge, jedenfalls einem Teil ihrer Länge, geteilt ausgebildet. Es ergibt sich ein an dem ersten Gehäuseteil 15, siehe etwa Figur 9, erster Teilbereich T1 bzw. zweiter Teilbereich T2 und ein an dem zweiten Gehäuseteil 16, siehe etwa Figuren 1 und 2, ausgebildeter dritter Teilbereich T3 bzw. vierter Teilbereich T4.

Während zwei Teilbereiche, etwa die Teilbereiche T1 und T2, als für sich gesehen zu einem Luftführungskanal nicht gehörend erkennbare Flächenbereiche einer ansonsten darüber hinausgehenden vorzugsweise ebenflächigen Oberfläche gegeben sein können, können die weiteren Teilbereiche T3 und T4 kammerartig gebildet sein, bei denen gleichsam im Öffnungszustand ein Deckel oder Boden fehlt. Durch die Zusammenwirkung im geschlossenen Zustand ergibt sich - erst - jeweils der geschlossene erste oder zweite Luftführungskanal 6, 7.

Die genannten Teilbereiche T3 und T4 sind durch ein Verschwenken des zweiten Gehäuseteiles 16 in eine bevorzugt verrastete Stellung, jedenfalls aber eine Anlagestellung, zu dem ersten Gehäuseteil zu dem jeweiligen benutzungsfähigen ersten bzw. zweiten Luftführungskanal 6, 7 ergänzbar. Hierbei kann zugleich die Trennwandung 10 relativ zu der Kammer 5 in eine Anlagestellung bzw. eine Freigabestellung bewegbar sein. Eine Begrenzungswandung der Teilbereiche T1, T2 und/oder T3, T4 kann auch mit einer Dichtungsausbildung, vorzugsweise aus einem Weichwerkstoff, versehen sein.

Eine Einstecheinrichtung E für die Kammer 5, zum Durchstechen der Abdeckung 13, kann zwei Einstechvorsprünge 17 aufweisen. Die Einstechvorsprünge 17 sind gegen Federkraft einer sich am Vorrichtungsgehäuse abstützenden Feder 19 in die Einstechstellung niederdrückbar. Nach Aufhebung einer Beaufschlagung etwa durch einen Finger eines Benutzers gehen sie in ihre Ausgangsstellung zurück.

Die Einstechvorsprünge 17 sind bevorzugt jeweils auf einer der Seiten der Trennwandung 10 ausgebildet. Während eine durch einen Einstechvorsprung 17 auf einer Seite der Trennwandung 10 gebildete Öffnung in der Abdeckung 13 der Kammer 5 dem zweiten Luftführungskanal 7 zugeordnet ist, ist die andere Öffnung dem ersten Luftführungskanal 6 zugeordnet.

Die Einstecheinrichtung E kann, wie dargestellt, an dem Betätigungselement 14 befestigt sein, entsprechend zusammen mit diesem niederdrückbar bzw. durch die Federkraft rückstellbar.

In der geschlossenen Stellung der Vorrichtung 1 ist bei eingesetzter Kammer 5, mit Ausnahme des kurzschlussartig wirkenden Verbindungskanals 8, durch die dichtend auf der Abdeckung 13 aufsitzenden Trennwandung 10 eine Unterbrechung des Haupt-Luftströmungsweges erreicht. Diese Unterbrechung ist erst durch Ausbildung der Öffnungen in der Kammer-Abdeckung 13 aufgehoben. Die Kammerhöhlung 12 wirkt hiernach als den ersten und den zweiten Luftführungskanal verbindender Kanalabschnitt.

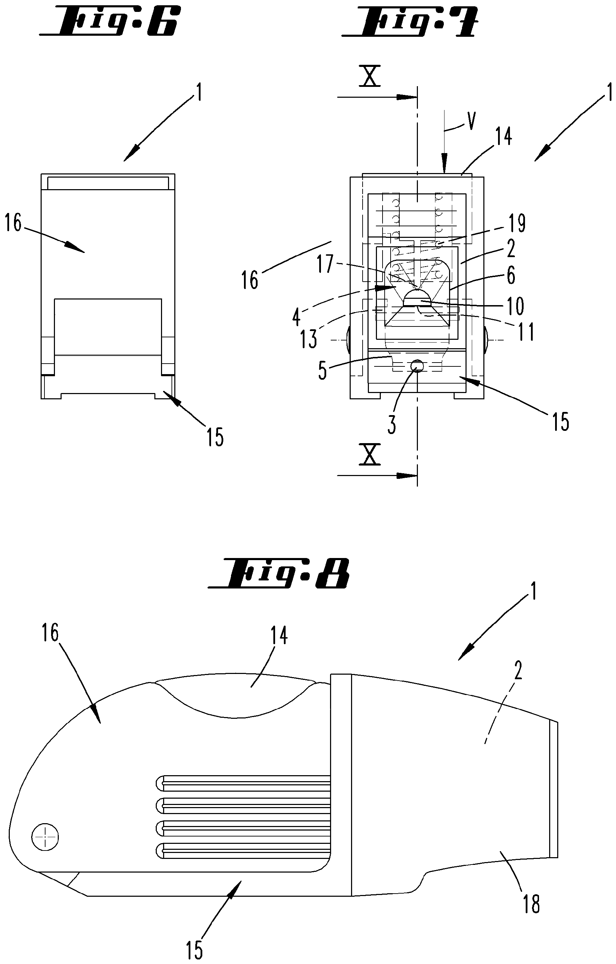

Zur Benutzung der Vorrichtung 1 wird diese zunächst in einen aufgeklappten Zustand gemäß Figur 1 versetzt. Die Kammer 5 wird in den Entleerungsbereich 4 eingesetzt, sodann werden durch Verschwenkung die Vorrichtungsteile zueinander geführt, in eine Stellung gemäß Figur 4 bzw. Figur 8. Durch Niederdrücken der Einstecheinrichtung E wird ein Benutzer sodann die erforderlichen Öffnungen in der Abdeckung 13 der Kammer 5 erzeugen und nach Loslassen durch Ansaugen an der Austragsdüse 2 einen Inhalationsvorgang durchführen. Hierdurch wird Luft durch den Lufteinlass 3 eingesaugt und durch den zweiten Luftführungskanal 7, wobei eine Führung in zwei übereinanderliegenden Etagen erfolgt, mit einem Verbindungsbereich bevorzugt nahe einer Scharnierverbindung 20 der Gehäuseteile 15 und 16, bis zu dem Entleerungsbereich 4 geführt. In dem Verbindungsbereich erfolgt eine Umlenkung der Luftströmung im zweiten Luftführungskanal 7 um bevorzugt 180°, so dass die Strömungsrichtung nach der Umlenkung entgegengesetzt gerichtet ist zu der Luftströmung vor der Umlenkung.

In dem Entleerungsbereich 4 strömt die Luft durch eine erste Öffnung in der Abdeckung 13 der Kammer 5 in die Kammer 5 ein, durchströmt die Kammerhöhlung 12 und strömt an der anderen Öffnung in den ersten Luftführungskanal 6 aus und von dort, als mit Substanz befrachtete Luft, bis zu der Austragsdüse 2 und ggf. in den Mund eines Nutzers.

Bevor die mit Substanz befrachtete Luft austritt, strömt durch den Verbindungskanal 8 aus dem zweiten Luftführungskanal 7 zusätzlich Luft in den ersten Luftführungskanal 6, nämlich eine Luft, die den Entleerungsbereich 4 nicht durchsetzt hat. Hierdurch ergibt sich eine günstige Verwirbelung und weiterer Aufschluss der Substanz in der Luft, die letztlich von einem Nutzer eingesaugt wird.

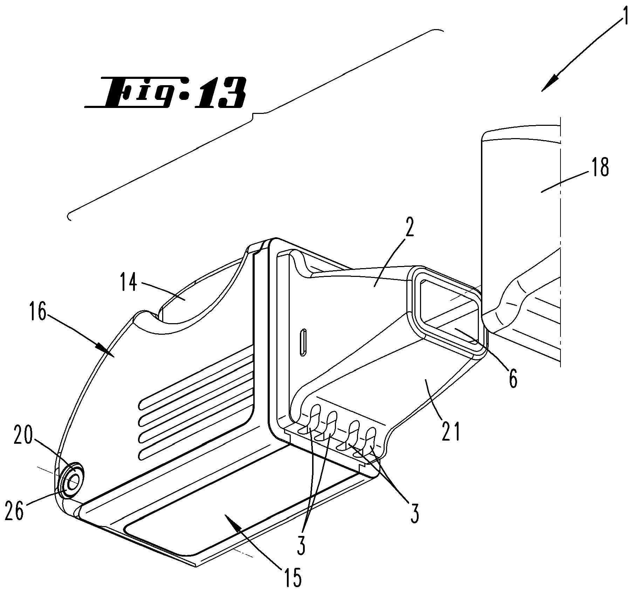

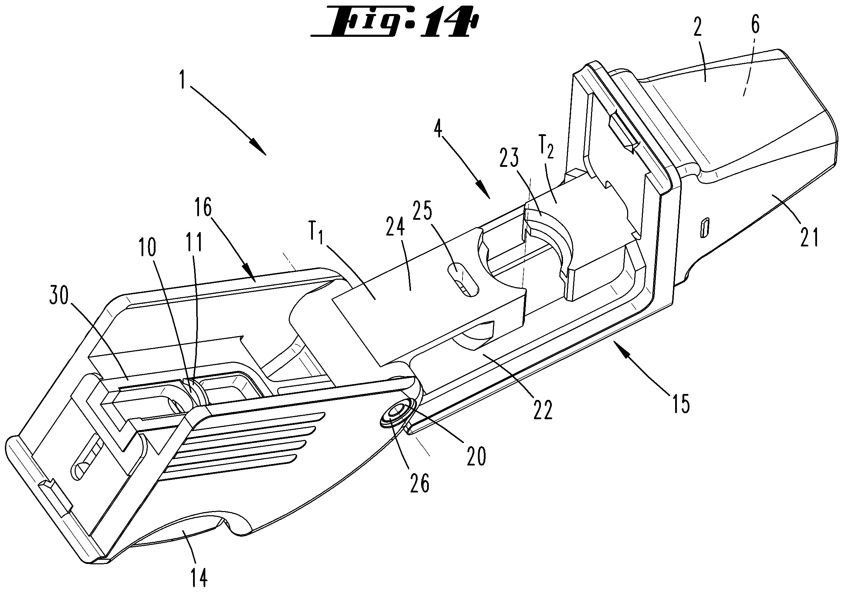

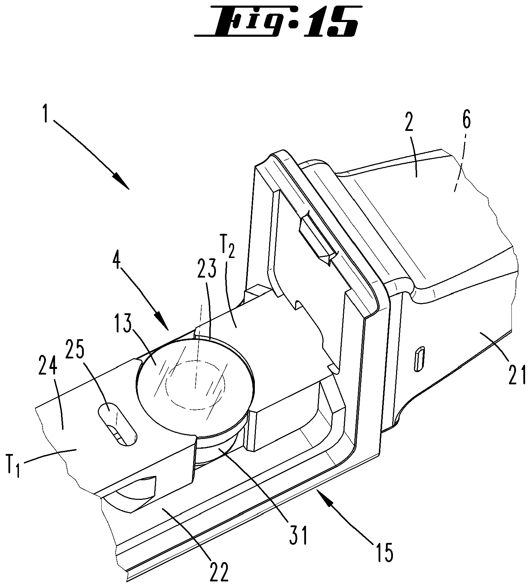

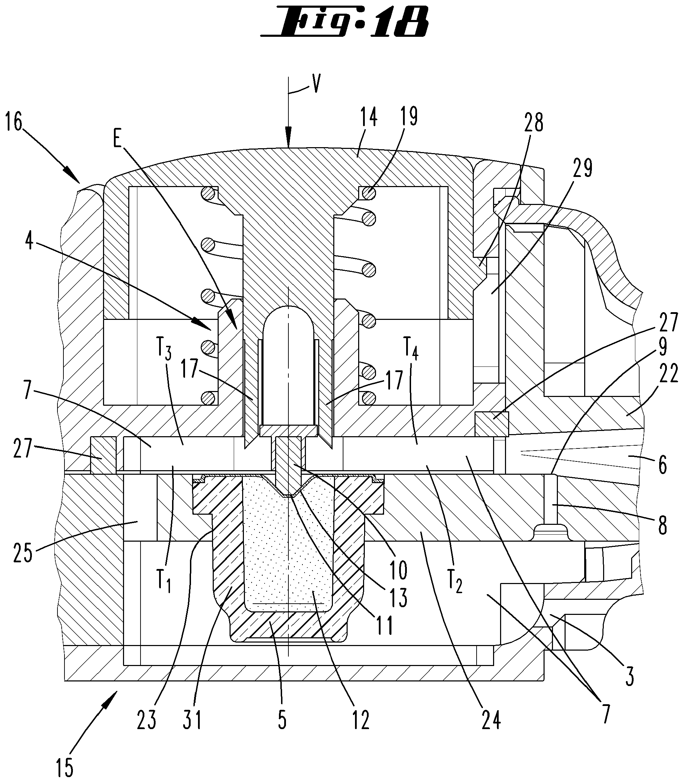

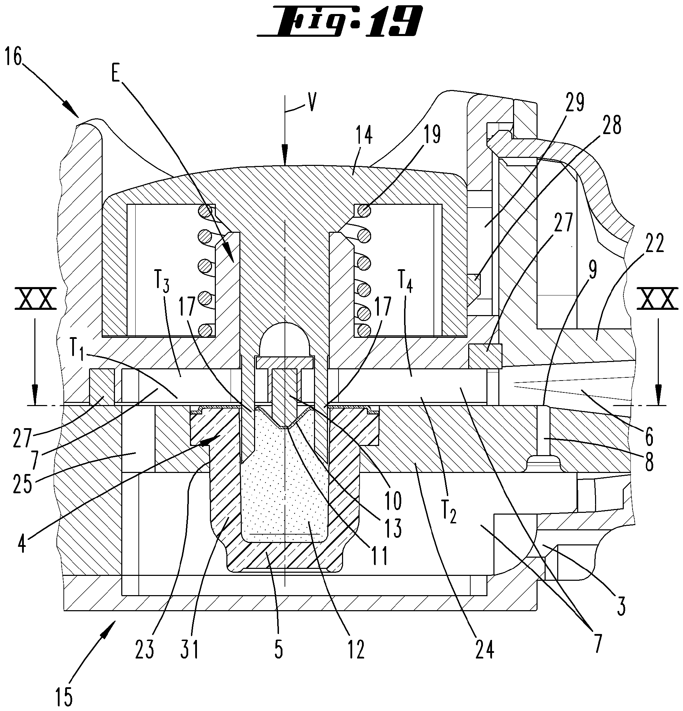

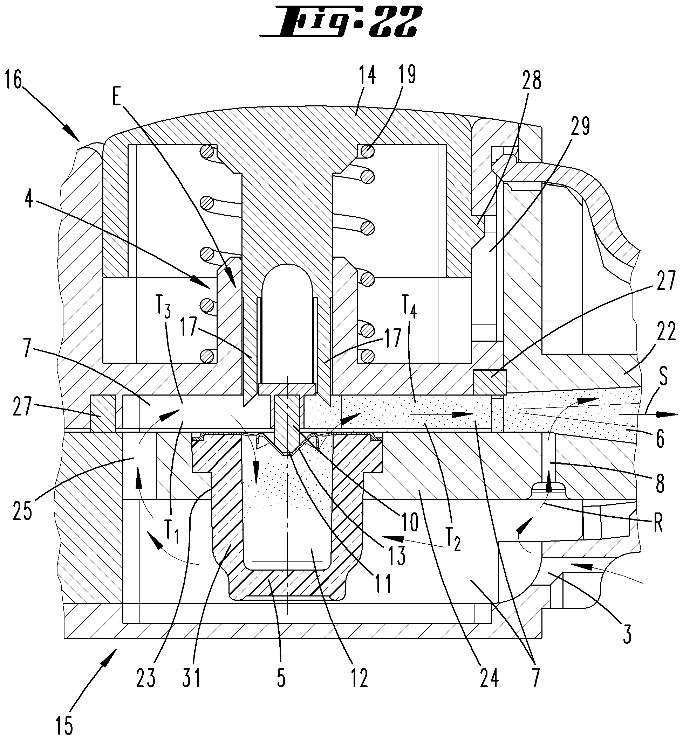

Die Darstellungen in den Figuren 13 bis 22 zeigen die Vorrichtung in einer zweiten Ausführungsform.

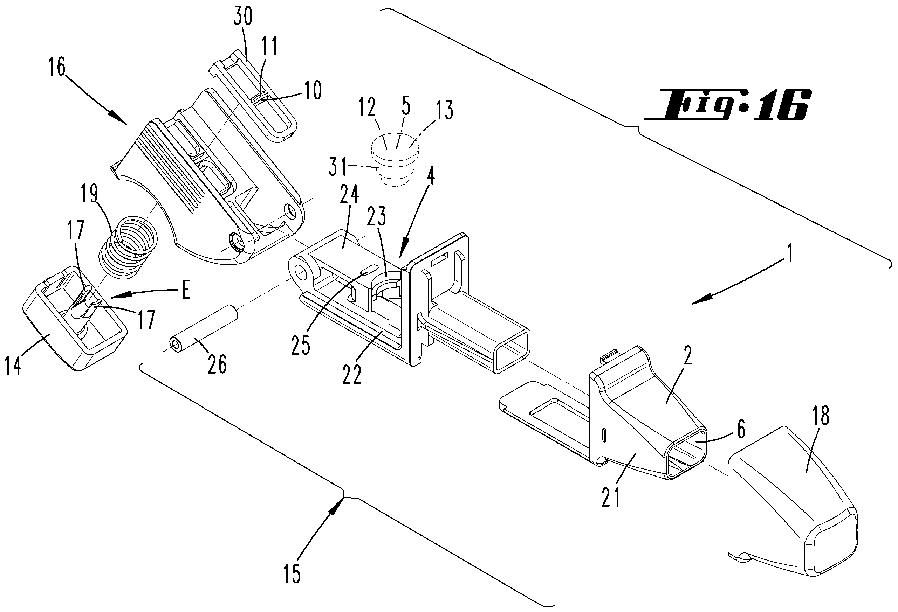

Wie insbesondere aus der perspektivischen Explosionsdarstellung in Figur 16 zu erkennen, setzt sich das erste Gehäuseteil 15 im Wesentlichen aus einem Düsenteil 21 und einem die Kammeraufnahme 23 aufweisenden Einsatzteil 22 zusammen. Die Kammeraufnahme 23 ergibt sich in einer im Nutzungszustand die Luftführungskanalabschnitte vor und nach der Umlenkung teilenden Einsatzteildecke 24. Diese ist endseitig des sich im Nutzungszustand ergebenden Luftführungskanals 7, zugewandt dem Scharnierbereich, von einer Durchströmungsöffnung 25 durchsetzt.

Das Einsatzteil 22 kann an bzw. in dem Düsenteil 21 rastgehaltert sein.

Unterseitig der mundstückartigen Austragsdüse 2 sind in dem dargestellten Ausführungsbeispiel in der Düsenteilwandung vier quer zur Luftströmungsrichtung nebeneinander angeordnete Lufteinlässe 3 ausgebildet.

Das zweite Gehäuseteil 16 ist über einen Zapfen 26 zur Bildung der Scharnierverbindung 20 an dem ersten Gehäuseteil 15 schwenkbeweglich gehaltert.

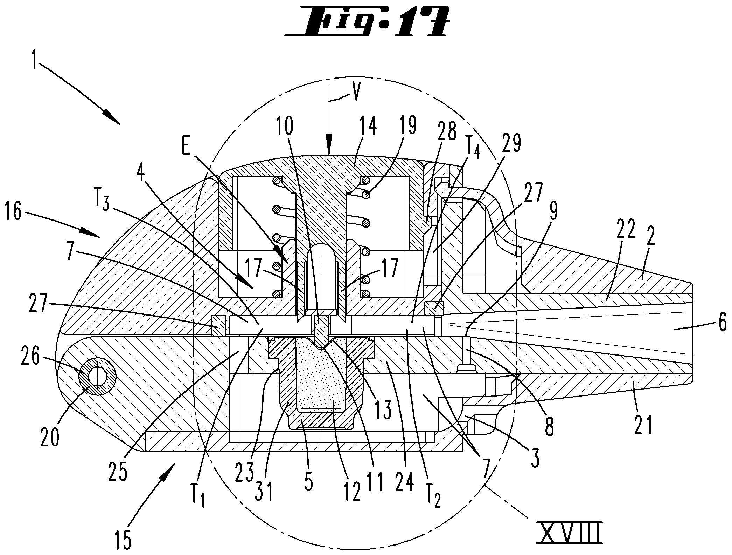

Im Bereich der mit dem ersten Gehäuseteil 15 korrespondierenden Flächen bzw. Flächenabschnitte, die zusammen mit dem ersten Gehäuseteil 15 insbesondere den Luftführungskanal 7 in Strömungsrichtung vor und hinter der Kammeraufnahme 23 begrenzen, sind Dichtungen 27 vorgesehen. Diese können an dem abschwenkbaren zweiten Gehäuseteil 16 gehaltert sein, zur Zusammenwirkung mit korrespondierenden Flächen des ersten Gehäuseteiles 15 in der Nutzungsstellung der Vorrichtung 1 (vgl. insbesondere Figur 17).

Auch in der zweiten Ausführungsform ist in dem zweiten Gehäuseteil 16 das Betätigungselement 14 schiebeverlagerbar gehaltert. Zur anschlagbegrenzten Verlagerbarkeit in Entspannungsrichtung der Feder 19 und somit zur unverlierbaren Fesselung, ggf. auch zur drehgesicherten Halterung des Betätigungselementes 14 in dem zweiten Gehäuseteil 16, greift eine wandungsaußenseitig an dem Betätigungselement 14 vorgesehene Nase 28 in einen sich in Niederdrückrichtung erstreckenden Führungsschlitz 29 des zweiten Gehäuseteils 16.

Die Trennwandung 10 kann gemäß der zweiten Ausführungsform, wie bspw. in Figur 16 dargestellt, an einem in das zweite Gehäuseteil 16 rastend einsetzbaren Rahmenteil 30 ausgebildet sein.

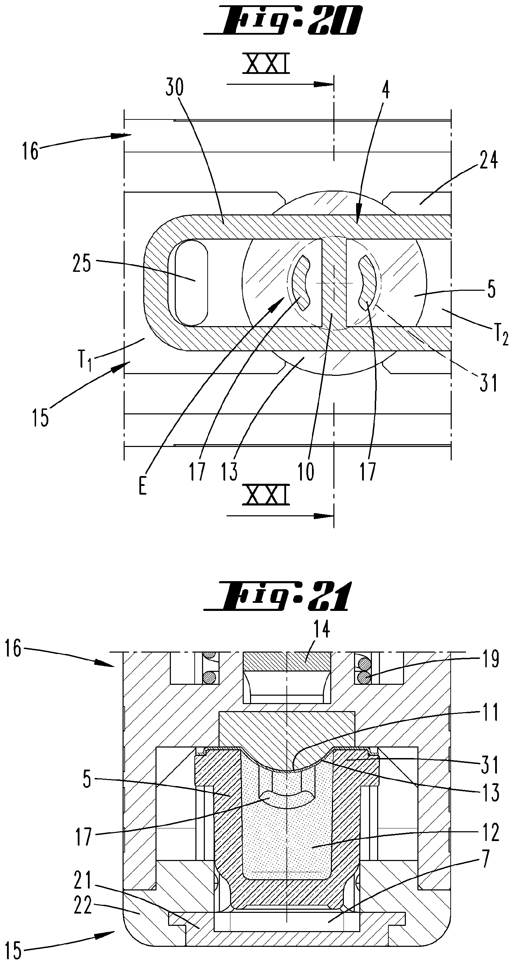

Wie insbesondere aus der Schnittdarstellung in Figur 21 zu erkennen, ist die Trennwandung 10 im Bereich der Stirnfläche 11 mit Bezug auf die Erstreckungsrichtung der Trennwandung 10 in Niederdrückrichtung vorgewölbt ausgebildet, insbesondere konvex gewölbt, weiter insbesondere kreisabschnittförmig. Dieser vorgewölbte Bereich wirkt bei geschlossener Vorrichtung 1, d.h. bei eingeschwenktem zweiten Gehäuseteil 16, derart auf die Abdeckung 13 der eingelegten Kammer 5 ein, dass diese sich konkav nach innen in Richtung auf die Kammerhöhlung 12 einwölbt (vgl. Figur 21). Die Einwölbung der Kammerabdeckung 13 kann ermöglicht sein zufolge entsprechend elastischer Ausgestaltung der Abdeckung 13, weiter insbesondere bei Ausbildung derselben als Folienabdeckung.

Die Trennwandung 10 kann sich in einer möglichen Ausgestaltung gemäß der Darstellung in Figur 21 mit Bezug auf den dargestellten Querschnitt beidseitig endseitig auf der Kammerwandung 31 und im Querschnitt bevorzugt vollflächig auf der zugewandten konvex gewölbten Abdeckung 13 abstützen.

Die Innenkontur der Kammerwandung 31 kann, wie in Figur 20 dargestellt, kreisrund gewählt sein, zur Bildung einer kreiszylinderförmigen Kammerhöhlung 12.

Die Einstechvorsprünge 17 erstrecken sich auch in der zweiten Ausführungsform mit Bezug auf einen Querschnitt quer zur Niederdrückrichtung V gemäß Figur 20 beidseitig der Trennwandung 10.

In diesem Querschnitt weisen die Einstechvorsprünge 17 eine der Innenkontur der Kammerwandung 31 angepasste Kontur, insbesondere Außenkontur auf. Entsprechend ergibt sich im Querschnitt eine Außenkontur in Form eines Kreislinienabschnittes. Insgesamt kann sich insbesondere spitzenseitig eines jeden Einstechvorsprunges 17 eine spitzschaufelartige Ausgestaltung ergeben, die eine günstige schneidende Durchsetzung der Kammerabdeckung 13 ermöglicht.

**Liste der Bezugszeichen**

| | | | |
|---|---|---|---|
| 1 | Vorrichtung | 28 | Nase |
| 2 | Austragsdüse | 29 | Führungsschlitz |
| 3 | Lufteinlass | 30 | Rahmenteil |
| 4 | Entleerungsbereich | 31 | Kammerwandung |
| 5 | Kammer | | |
| 6 | Luftführungskanal | | |
| 7 | Luftführungskanal | | |
| 8 | Verbindungskanal | E | Einstecheinrichtung |
| 9 | Einströmmündung | R | Einströmrichtung |
| 10 | Trennwandung | S | Strömungsrichtung |
| 11 | Stirnfläche | T1 | erster Teilbereich |
| 12 | Kammerhöhlung | T2 | zweiter Teilbereich |
| 13 | Abdeckung | T3 | dritter Teilbereich |
| 14 | Betätigungselement | T4 | vierter Teilbereich |
| 15 | erstes Gehäuseteil | V | Niederdrückrichtung |
| 16 | zweites Gehäuseteil | | |
| 17 | Einstechvorsprung | | |
| 18 | Kappe | | |
| 19 | Feder | | |
| 20 | Scharnierverbindung | | |
| 21 | Düsenteil | | |
| 22 | Einsatzteil | | |
| 23 | Kammeraufnahme | | |
| 24 | Einsatzteildecke | | |
| 25 | Durchströmöffnung | | |
| 26 | Zapfen | | |
| 27 | Dichtung | | |

## Patentansprüche

1. Vorrichtung (1) zur Ausgabe einer durch Luft austragbaren Substanz mit einem Lufteinlass (3), einer Austragsdüse (2), durch welche mit Substanz befrachtete Luft austreten kann, einem Entleerungsbereich (4), in welchem eine Kammer (5) mit auszutragender Substanz in einer Kammeraufnahme angeordnet ist, einem von dem Entleerungsbereich (4) zu der Austragsdüse (2) führenden ersten Luftführungskanal (6), einem von dem Lufteinlass (3) zu dem Entleerungsbereich (4) führenden zweiten Luftführungskanal (7), wobei in dem Entleerungsbereich (4) zwischen dem ersten und dem zweiten Luftführungskanal (6, 7) eine Trennwandung (10) ausgebildet ist und die Trennwandung (10) aus einem Hartwerkstoff besteht, wobei weiter eine der Kammer (5) zugewandte freie Stirnfläche (11) der Trennwandung (10) mit einem Weichwerkstoff versehen ist, die Kammer (5) in einer Überdeckung zu einer Substanz enthaltenden Kammerhöhlung (12) eine Folienabdeckung(13) aufweist und die Trennwandung (10) vermittels dieses Weichwerkstoffes dichtend an der Folienabdeckung anliegt.

2. Vorrichtung nach Anspruch 1, **dadurch gekennzeichnet, dass** der zweite Luftführungskanal (7) über einen Verbindungskanal (8) mit dem ersten Luftführungskanal (6) in einer Strömungsverbindung ist, mit einer Einströmrichtung (R) von durch eine Einströmmündung (9) durch den Verbindungskanal (8) in den ersten Luftführungskanal (6) strömender Luft, die senkrecht gerichtet ist zu einer Strömungsrichtung (S) der mit Substanz befrachteten Luft in den ersten Luftführungskanal (6) im Bereich der Einströmmündung (9).

3. Vorrichtung (1) nach einem der vorhergehenden Ansprüche, **dadurch gekennzeichnet, dass** die Kammeraufnahme in einem ersten Gehäuseteil (15) ausgebildet ist und ein zweites Gehäuseteil (16) vorgesehen ist, das mit dem ersten Gehäuseteil (15) verschwenkbar verbunden ist, und dass der Luftführungskanal (6, 7) über einen Teil seiner Länge längsgeteilt ausgebildet ist, mit an dem ersten und dem zweiten Gehäuseteil (15,16) ausgebildeten Teilbereichen (T1, T2, T3, T4).

4. Vorrichtung (1) nach einem der vorhergehenden Ansprüche, **dadurch gekennzeichnet, dass** eine Einstecheinrichtung (E) zur Ausbildung einer Einstechöffnung in der Folienabdeckung vorgesehen ist, und dass weiter die Einstecheinrichtung (E) zwei Einstechvorsprünge (17) aufweist, von denen einer zugeordnet der einen Seite der Trennwandung (10) und der andere zugeordnet der anderen Seite der Trennwandung (10) ausgebildet ist.

5. Vorrichtung nach einem der Ansprüche 2 bis 4, **dadurch gekennzeichnet, dass** die Einströmmündung (9) schlitzartig gestaltet ist und sich über mehr als 50% einer quer zu der Strömungsrichtung (S) betrachteten Breite des ersten Luftströmungskanals (6) erstreckt.

6. Vorrichtung (1) nach einem der Ansprüche 3 bis 5, **dadurch gekennzeichnet, dass** an dem zweiten Gehäuseteil (16) ein dritter Teilbereich (T3) des zweiten Luftführungskanals (7) und ein vierter Teilbereich (T4) des ersten Luftführungskanals (6) gegeben ist, wobei weiter der dritte Teilbereich (T3) und der vierte Teilbereich (T4) durch ein Verschwenken des zweiten Gehäuseteils (16) in eine bevorzugt verrastete, jedenfalls aber eine Anlagestellung, zu dem ersten Gehäuseteil (15) zu dem jeweiligen ersten bzw. zweiten Luftkanal (6, 7) ergänzbar sind.

7. Vorrichtung nach Anspruch 6, **dadurch gekennzeichnet, dass** bei dem Verschwenken zugleich die für die Luftströmung undurchlässige Trennwand (10) bei eingesetzter Kammer (5) relativ zu der Kammer (5) in eine Anlagestellung bewegbar ist, dass durch die Trennwand (10) eine unmittelbare Luftströmung von dem zweiten Luftführungskanal (7) in den ersten Luftführungskanal (6) unterbunden ist und dass die Luftströmung im Benutzungszustand nur durch die Kammer (5) verläuft.

8. Vorrichtung (1) nach einem der Ansprüche 4 bis 7, **dadurch gekennzeichnet, dass** ein Einstechvorsprung (17) in einem Querschnitt an eine in einer Niederdrückrichtung (V) gesehen gegebene Innenkontur einer Kammerwandung (31) angepasst ist, dass die Kammerwandung (31) in dem Querschnitt kreisringförmig gebildet ist und der Einstechvorsprung (17) in einem die Kammerabdeckung (13) bei einer Verlagerung des Einstechvorsprungs (17) in einer Einstechrichtung durchsetzenden Bereich eine kreisabschnittsförmige Außenkontur aufweist, und dass der Einsteckvorsprung (17) eine Spitze oder Schneide aufweist, die im Zuge des Einstechens unmittelbar an einer Innenwand der Kammer (5) entlangfährt.

9. Vorrichtung nach einem der vorhergehenden Ansprüche, **dadurch gekennzeichnet, dass** sich die Trennwandung (10) mit ihrer freien Stirnfläche in Anlagestellung zu der Folienabdeckung quergerichtet zu der Strömungsrichtung (S) im Luftführungskanal erstreckt.

10. Vorrichtung nach einem der Ansprüche 4 bis 9, **dadurch gekennzeichnet, dass** die Einstecheinrichtung (E) entgegen einer Rückstellkraft verlagerbar ist, zur Durchsetzung der Folienabdeckung der Kammer (5) durch die Einstechvorsprünge (17), wobei, bevorzugt, die Rückstellkraft aus einer Feder (19) resultiert.

11. Vorrichtung nach einem der Ansprüche 4 bis 10, **dadurch gekennzeichnet, dass** die Vorrichtung (1) ein verlagerbares Betätigungselement (14) aufweist, zur Einwirkung auf die Einstecheinrichtung (E).

12. Vorrichtung nach einem der Ansprüche 4 bis 11, **dadurch gekennzeichnet, dass** nach einem Durchstechen der Folienabdeckung durch die Einstechvorsprünge (17) die Kammer (5) den ersten Luftführungskanal (6) mit dem zweiten Luftführungskanal (7) verbindet.

13. Vorrichtung nach einem der Ansprüche 3 bis 12, **dadurch gekennzeichnet, dass** das zweite Gehäuseteil (16) über eine Scharnierverbindung (20) mit dem ersten Gehäuseteil (15) verbunden ist und dass eine Umlenkung des zweiten Luftführungskanals (7) zugeordnet der Scharnierverbindung (20) ausgebildet ist.

14. Vorrichtung nach Anspruch 13, **dadurch gekennzeichnet, dass** in Strömungsrichtung nach der Umlenkung die Luftströmung entgegengesetzt gerichtet ist zu der Luftströmung vor der Umlenkung.

15. Vorrichtung nach einem der vorhergehenden Ansprüche, **dadurch gekennzeichnet, dass** die Folienabdeckung (13) der Kammer (5) in einer Ansicht von außen konkav verlaufend ausgebildet ist.

## Claims

1. The device (1) for dispensing an air-dischargeable substance, having an air inlet (3), a discharge nozzle (2) through which substance-loaded air can be discharged, an outflow region (4), in which a chamber (5) containing substance to be discharged can be arranged in a chamber receptacle, a first air guide duct (6) leading from the outflow region (4) to the discharge nozzle (2), a second air guide duct (7) leading from the air inlet (3) to the outflow region (4), wherein a partition (10) is formed in the outflow region (4) between the first and second air guide ducts (6, 7), and the partition (10) is made of a hard material, wherein, furthermore, a free end face (11) of the partition (10) facing the chamber (5) is provided with a soft material, the chamber (5) has a film cover (13) in a cover for a chamber cavity (12) containing substance, and the partition (10) is in sealing contact with the film cover by means of this soft material.

2. The device according to claim 1, **characterized in that** the second air guide duct (7) is also fluidically connected by a connecting duct (8) to the first air guide duct (6), and with an inflow direction (R) of air flowing through an inflow opening (9) and through the connecting duct (8) into the first air guide duct (6), said air being directed perpendicular to the direction of flow (S) of the substance-loaded air into the first air guide duct (6) in the region of the inflow opening (9).

3. The device (1) according to one of the preceding claims, **characterized in that** the chamber receptacle is designed in a first housing part (15), and a second housing part (16), which is pivotably connected to the first housing part (15), is provided for, and that the air guide duct (6, 7) is designed with longitudinal divisions over a portion of its length, having subregions (T1, T2, T3, T4) formed on the first and second housing parts (15, 16).

4. The device (1) according to one of the preceding claims, **characterized in that** a puncture device (E) is provided for forming a puncture opening in the film cover, and that further the puncture device (E) additionally has two puncture protrusions (17), one of which is designed to be associated with one side of the partition (10) and the other of which is designed to be associated with the other side of the partition (10).

5. The device according to one of the claims 2 to 4, **characterized in that** the inflow opening (9) is designed with a slot, and extend over more than 50 % of a width of the first air flow duct (6), as seen across the direction of flow (S).

6. The device (1) according to one of the claims 3 to 5, **characterized in that** a third subregion (T3) of the second air guide duct (7) and a fourth subregion (T4) of the first air guide duct (6) are provided for on the second housing part (16), wherein the third subregion (T3) and the fourth subregion (T4) can further be supplemented by pivoting the second housing part (16) into a preferably locked position, but at any rate a contact position with the first housing part (15) to the respective first respectively second air duct (6, 7).

7. The device according to claim 6, **characterized in that** when pivoting at the same time, a partition (10) which is impermeable for the air flow can be moved into a contact position with the chamber (5) inserted, relative to the chamber (5), a direct air flow from the second air guide duct (7) into the first air guide duct (6) is suppressed by the partition (10), and the air flow runs only through the chamber (5) when in use.

8. The device (1) according to one of the claims 4 to 7, **characterized in that** a puncture protrusion (17) is adapted in a cross section to an inside contour of a chamber wall (31) as seen in a hold-down direction (V), that the chamber wall (31) has a cross section in the form of a circular ring, and the puncture protrusion (17) has an outside contour in the form of a segment of a circle in a region puncturing the chamber cover (13) in displacement of the puncture protrusion (17) in a puncture direction, and that the puncture protrusion (17) has a tip or a cutting edge which slides directly along an inside wall of the chamber (5) as part of the puncturing.

9. The device according to one of the preceding claims, **characterized in that** the partition (10) extends transversely to the direction of flow (S) in the air guide duct, with its free end face in a position in which it is in contact with the film cover.

10. The device according to one of the claims 4 to 9, **characterized in that** the puncture device (E) is displaceable against a restoring force, for puncturing the film cover of the chamber (5) by means of the puncture protrusions (17), wherein, preferably the restoring force results from a spring (19).

11. The device according to one of the claims 4 to 10, **characterized in that** the device (1) has a displaceable actuating element (14) for acting on the puncture device (E).

12. The device according to one of the claims 4 to 11, **characterized in that** after the film cover has been punctured by the puncture protrusions (17), the chamber (5) connects the first air guide duct (6) to the second air guide duct (7).

13. The device according to one of the claims 3 to 12, **characterized in that** the second housing part (16) is connected by a hinge connection (20) to the first housing part (15), and a deflection in the second air guide duct (7) is designed to be associated with the hinge connection (20).

14. The device according to claim 13, **characterized in that** the air flow in the direction of flow downstream from the deflection is preferably directed in the opposite direction from the air flow upstream from the deflection.

15. The device according to one of the preceding claims, **characterized in that** the film cover (13) of the chamber (5) is designed to be run in a concave shape in a view from the outside.

## Revendications

1. Dispositif (1) pour la distribution d'une substance pouvant être évacuée par de l'air, comprenant une entrée d'air (3), une buse d'évacuation (2) par laquelle de l'air chargé de substance peut sortir, une zone de vidange (4) dans laquelle une chambre (5) contenant la substance à distribuer est agencée dans un logement de chambre, un premier canal de guidage d'air (6) menant de la zone de vidange (4) à la buse d'évacuation (2), un deuxième canal de guidage d'air (7) menant de l'entrée d'air (3) à la zone de vidange (4), dans lequel une paroi de séparation (10) est formée dans la zone de vidange (4) entre le premier et le deuxième conduit de guidage d'air (6, 7) et la paroi de séparation (10) est constituée d'un matériau dur, dans lequel en outre une surface frontale libre (11) de la paroi de séparation (10) tournée vers la chambre (5) est pourvue d'un matériau mou, la chambre (5) comporte un opercule (13) qui recouvre une cavité de chambre (12) contenant une substance et la paroi de séparation (10) s'appuie de manière étanche contre l'opercule par le biais de ce matériau mou.

2. Dispositif selon la revendication 1, **caractérisé en ce que** le deuxième conduit de guidage d'air (7) est en liaison d'écoulement avec le premier conduit de guidage d'air (6) par l'intermédiaire d'un conduit de liaison (8), avec une direction d'entrée (R) de l'air s'écoulant par une ouverture d'entrée (9) à travers le conduit de liaison (8) dans le premier conduit de guidage d'air (6), qui est dirigée perpendiculairement à une direction d'écoulement (S) de l'air chargé de substance dans le premier conduit de guidage d'air (6) dans la région de l'ouverture d'entrée (9).

3. Dispositif (1) selon l'une des revendications précédentes, **caractérisé en ce que** le logement de chambre est situé dans une première partie de boîtier (15) et une deuxième partie de boîtier (16) est prévue qui est reliée de manière pivotante à la première partie de boîtier (15), et **en ce que** le conduit de guidage d'air (6, 7) est divisé longitudinalement sur une partie de sa longueur avec des régions partielles (T1, T2, T3, T4) formées au niveau de la première et de la deuxième partie de boîtier (15, 16).

4. Dispositif (1) selon l'une des revendications précédentes, **caractérisé en ce qu'**un dispositif de perçage (E) est prévu pour former une ouverture de perçage dans l'opercule, et **en ce que** le dispositif de perçage (E) présente en outre deux saillies de perçage (17) dont l'une est formée en étant associée à un côté de la paroi de séparation (10) et l'autre à l'autre côté de la paroi de séparation (10).

5. Dispositif selon l'une des revendications 2 à 4, **caractérisé en ce que** l'ouverture d'entrée (9) est conçue en forme de fente et s'étend sur plus de 50% d'une largeur du premier canal d'écoulement d'air (6) considérée transversalement à la direction d'écoulement (S).

6. Dispositif (1) selon l'une des revendications 3 à 5, **caractérisé en ce qu'**une troisième région partielle (T3) du deuxième canal de guidage d'air (7) et une quatrième région partielle (T4) du premier canal de guidage d'air (6) sont prévues au niveau de la deuxième partie de boîtier (16), dans lequel en outre la troisième région partielle (T3) et la quatrième région partielle (T4) peuvent être complétées en faisant pivoter la deuxième partie du boîtier (16) dans une position de préférence encliquetée, mais en tout cas de contact, avec la première partie du boîtier (15) pour former les premier et deuxième conduits d'air respectifs (6, 7) .

7. Dispositif selon la revendication 6, **caractérisé en ce que** lorsque la chambre (5) est insérée, la paroi de séparation (10) imperméable à l'écoulement de l'air peut être déplacée dans une position de contact par rapport à la chambre (5) simultanément au mouvement de pivotement, **en ce que** la paroi de séparation (10) empêche un écoulement d'air direct du deuxième conduit de guidage d'air (7) dans le premier conduit de guidage d'air (6) et **en ce que**, dans l'état d'utilisation, l'écoulement d'air ne traverse que la chambre (5).

8. Dispositif (1) selon l'une des revendications 4 à 7, **caractérisé en ce qu'**une saillie de perçage (17) est adaptée en section transversale à un contour intérieur donné d'une paroi de chambre (31) vu dans une direction de pressage vers le bas (V), **en ce que** la paroi de chambre (31) est réalisée en section transversale en forme d'anneau circulaire et que la saillie de perçage (17) présente un contour extérieur en forme de partie circulaire dans une zone qui traverse l'opercule de chambre (13) lors d'un déplacement de la saillie de perçage (17) dans une direction de perçage, et **en ce que** la saillie de perçage (17) présente une pointe ou une arête de coupe qui passe directement le long d'une paroi intérieure de la chambre (5) au cours du perçage.

9. Dispositif selon l'une des revendications précédentes, **caractérisé en ce que** la paroi de séparation (10) avec sa surface frontale libre en position d'appui contre l'opercule s'étend dans une direction transversale à la direction d'écoulement (S) dans le conduit de guidage d'air.

10. Dispositif selon l'une des revendications 4 à 9, **caractérisé en ce que** le dispositif de perçage (E) est déplaçable contre une force de rappel, pour le passage des saillies de perçage (17) à travers l'opercule de la chambre (5), la force de rappel résultant de préférence d'un ressort (19).

11. Dispositif selon l'une des revendications 4 à 10, **caractérisé en ce que** le dispositif (1) présente un élément d'actionnement (14) déplaçable pour agir sur le dispositif de perçage (E).

12. Dispositif selon l'une des revendications 4 à 11, **caractérisé en ce que**, après un percement de l'opercule par les saillies de perçage (17), la chambre (5) relie le premier conduit de guidage d'air (6) au deuxième conduit de guidage d'air (7).

13. Dispositif selon l'une des revendications 3 à 12, **caractérisé en ce que** la deuxième partie de boîtier (16) est reliée à la première partie de boîtier (15) par une liaison à charnière (20) et qu'une déviation du deuxième canal d'air (7) est formée en association avec la liaison à charnière (20).

14. Dispositif selon la revendication 13, **caractérisé en ce que** dans la direction d'écoulement après la déviation, le flux d'air est dirigé à l'opposé du flux d'air avant la déviation.

15. Dispositif selon l'une des revendications précédentes, **caractérisé en ce que** l'opercule (13) de la chambre (5) s'étend de manière concave lorsqu'il est vu de l'extérieur.
